# EUROPÄISCHE PATENTANMELDUNG

(11) **EP 3 192 547 A1**
(43) Veröffentlichungstag der Anmeldung: **19.07.2017**
(21) Anmeldenummer: 17150016.8
(22) Anmeldetag: 02.01.2017
(51) Int. Cl.: A61M 5/315

(54) **KOLBENSTANGE FÜR MEDIZINISCHE PACKMITTEL**

(30) Priorität: 15.01.2016 DE 102016200434
(71) Anmelder: SCHOTT Schweiz AG, 9001 St. Gallen (CH)
(72) Erfinder: KOMANN, Christian, 9000 St. Gallen (CH)
(74) Vertreter: Sawodny, Michael-Wolfgang

(57) **Zusammenfassung**

Die Erfindung betrifft eine Kolbenstange, bevorzugt hohle Kolbenstange für medizinische Packmittel, insbesondere Spritzenkörper, Karpulen, wobei die Kolbenstange ein erstes und ein zweites Kolbenstangenende umfasst, dadurch gekennzeichnet, dass
die Kolbenstange eine Torsionsachse und Querschnittsflächen umfasst sowie Mittel zur Erhöhung der Biege und Torsionssteifigkeit
dadurch gekennzeichnet, dass
die Mittel zur Erhöhung der Biege- und Torsionssteifigkeit, Bestandteile der Kolbenstange, die wenigstens eine Querschnittsfläche entfernt zur Torsionsachse zur Verfügung stellen, sind.

## Beschreibung

Die Erfindung betrifft eine Kolbenstange für medizinische Packmittel, insbesondere Spritzenkörper oder Karpulen, wobei die Kolbenstange ein erstes und ein zweites Kolbenstangenende aufweist.

Bei Spritzen, wie sie beispielsweise aus der US 7,824,380 B2 bekannt sind, bestehen die Kolbenstangen aus gekreuzten Rippen. Bei der US 7,824,380 B2 wird das Kolbenstangenende, an dem der Kolbenstopfen befestigt wird, zusätzlich mit Auflagerippen versehen, um bei einem Verkanten der Kolbenstange das Lösen der Kolbenstange zu erleichtern. Ein Verkanten der Kolbenstange kann bereits bei minimal eingeschobener Kolbenstange in den Spritzenkörper auftreten. Die Kolbenstange im Stand der Technik, ist im Wesentlichen ein langes Strukturbauteil mit einer hohen Steifigkeit gegenüber Bedienkräften. Nachteilig ist allerdings, dass eine Kolbenstange im Stand der Technik, um den Werkzeugaufwand gering zu halten, nur über eine geringe Steifigkeit bei Torsionslasten verfügt. Ein weiterer Nachteil ist darin zu sehen, dass der Abstand zwischen den Auflagerippen zum Vermeiden von kritischem Verkanten sehr groß ist.

In der US 2,392,104 B ist eine Spritze mit einem Glaskolben gezeigt. Der Glaskolben gemäß der US 2,392,104 B kann hohl ausgebildet sein. Nachteilig an der aus Glas ausgebildeten hohlen Kolbenstange der US 2,392,104 B ist deren hohes Gewicht. Des Weiteren geht aus der US 2,392,104 B nicht hervor, wie eine Kolbenstange ausgebildet sein muss, damit eine ausreichende Steifigkeit bei Torsionslasten zur Verfügung gestellt wird.

Aus der DE 38 74 162 T2 ist eine Spritze mit Mitteln zum Schutz gegen Nadeleinstiche und Medikamentenmissbrauch bekannt geworden. Der Kolbenschaft aus der DE 38 74 162 T2 weist an der Außenseite Verriegelungsfallen auf, um eine Herausnahme des Schaftes aus dem distalen Ende des Spritzenzylinders zu verhindern.

Die DE 199 23 131 A1 zeigt eine medizinische Injektionsspritze. Die Spritze gemäß der DE 199 23 131 A1 ist als Kunststoffspritze ausgebildet und umfasst eine Kolbenstange mit einer speziellen Querschnittsformgebung, um die notwendige Steife der Kolbenstange zur Verfügung zu stellen. Die in der DE 199 23 131 A1 beschriebene Kolbenstange zeichnet sich durch eine hohe Festigkeit aufgrund der Steife aus. Im Gegensatz hierzu erfordert, aber eine hohe Biege- und Torsionssteifigkeit eine hohe Steifigkeit gegenüber Torsionslasten und keine hohe Festigkeit alleine.

Aus der US 5,032,114 A ist eine Spritze mit einem Kolben bekannt geworden, wobei der Kolben eine Helix umfasst. Die Aufgabe dieser helicalen Struktur ist aber an keiner Stelle der US 5,032,114 A beschrieben. Die spiralförmige Struktur wie in der US 5,032,114 A ist aber nicht steif hinsichtlich Torsion, weswegen auch die US 5,032,114 A keine Struktur zeigt, mit der die Torsionssteifigkeit erhöht wird.

Die DE 1 175 146 zeigt einen Gefäßverschluss mit federndem Abstandshalter, der eine helicale Struktur aufweist und keine Spritze.

Aufgabe der Erfindung ist es somit, die Nachteile des Standes der Technik zu überwinden.

Erfindungsgemäß wird diese Aufgabe dadurch gelöst, dass eine Kolbenstange insbesondere eine hohle Kolbenstange für medizinische Packmittel, insbesondere für Spritzenkörper oder Karpulen zur Verfügung gestellt wird, bei dem die Kolbenstange eine Torsionsachse und Querschnittsflächen umfasst sowie Mittel zur Erhöhung der Biege- und Torsionssteifigkeit aufweist. Die Mittel zur Erhöhung der Biege- und Torsionssteifigkeit sind Bestandteile der Kolbenstange, die wenigstens eine Querschnittsfläche, entfernt zur Torsionsachse aufweist oder bereitstellt.

Besonders bevorzugt ist es, wenn die Kolbenstange mit Hilfe eines Spritzgussverfahrens erhalten wird.

Wenn die Kolbenstange mit Hilfe eines Spritzgussverfahrens erhalten wird, so ist das Material der Kolbenstange bevorzugt ein Kunststoffmaterial. Besonders bevorzugte Kunststoffe sind thermoplastische Kunststoffe. Ein geeignetes Material ist Polyethylen (PE). Ein anderes Polymer kann Polypropylen (PP) oder Polystyrol (PS) oder Polyoxymethylen (POM) sein. Die Materialien der Kolbenstange sind Thermoplaste und unterscheiden sich von den Materialien des Spritzenkörpers. Die Materialien des Spritzenkörpers können im Gegensatz zur Kolbenstange mit einem in den Spritzenkörper abgefüllten pharmazeutischen Praxis für längere Zeit in Kontakt treten. Um pharmazeutische Produkte für einen längeren Zeitraum in dem Spritzenkörper lagern zu könnnen, ist es notwendig den Spritzenkörper aus weitgehend inerten Materialien, aus denen keine Stoffe in das im Spritzenkörper gelagerte pharmazeutische Produkt diffundieren können, zu verwenden. Geeignet hierfür sind hochwertige Kunststoffe wie COC und COP, die hart und auch relativ spröde sind. Um die Spritzenkörper zur Lagerung pharmazeutischer Produkte einsetzen zu können, ist es vorteilhaft, wenn die Materialien bei Temperaturen >100°C, insbesondere 121°C, insbesondere maximal 180°C sterilisierbar sind. Für Cycloolefinpolymere (COP) und Cycloolefincopolymere (COC) trifft dies zu. Als Material für den Spritzenkörper wäre auch Glas anstelle von COC und COP möglich. Ein weiteres mögliches Material für den Spritzenkörper wäre auch Polypropylen (PP) oder Polyethylen (PE). Polypropylen (PP) oder Polyethylen (PE) sind zwar weichere Materialien, die Spannungen aufnehmen können, allerdings sind Sie aufgrund ihres Diffusionverhaltens nur für eine kurzzeitige Lagerung pharmazeutischer Produkte geeignet.

Wird die Kolbenstange als Hohlkörper ausgebildet, so wird die mit Hilfe eines Spritzgussverfahrens hergestellte Kolbenstange mit Hilfe eines Spritzguss-Werkzeuges mit einem langen Kern hergestellt, den man beispielsweise von der Kolbenstopfenseite in die Kolbenstange einführt. Eine sich dann ergebende Kolbenstange ist nur einseitig geschlossen. Alternativ könnte auch mit Hilfe eines Gasinnendruck-Verfahrens aus einem Kunststoffmaterial ein geschlossener Hohlkörper erhalten werden. Bei dem Gasinnendruckverfahren handelt es sich um ein spezielles Spritzgussverfahren.

Durch die Verwendung eines Kunststoffmaterials für die Kolbenstange anstelle eines Glasmaterials wie in der US 2,392,104 B offenbart, können erhebliche Gewichtsreduktionen erreicht werden, insbesondere wenn auch die Kolbenstange als Kunststoffteil ausgeführt ist.

Damit die aus einem Thermoplasten bestehende Kolbenstange nicht mit dem in dem Spritzenkörper gelagerten pharmazeutischen Produkt in Kontakt kommt, ist bevorzugt vorgesehen, auf der Kolbenstange einen Stopfen vorzusehen. Der Stopfen kann beispielsweise aus Gummi bestehen. Üblicherweise weist der Stopfen eine Innengewinde auf und die Kolbenstange an ihrem distalen Ende eine Außengewinde, so dass die Kolbenstange in den Stopfen eingeschraubt werden kann.

In einer ersten Ausgestaltung der Erfindung ist vorgesehen, dass die Kolbenstange wenigstens abschnittsweise schräg gegenüber der Torsionsachse angeordnete Versteifungselemente, insbesondere in Form von Versteifungsrippen, als Mittel zur Erhöhung der Biege- und Torsionssteifigkeit umfasst. Besonders bevorzugt ist es, wenn die Versteifungselemente, insbesondere die Versteifungsrippen, gegenüber der Torsionsachse um einen Winkel α, der bevorzugt > 10°, insbesondere > 20°, bevorzugt > 30°, insbesondere 45° geneigt sind. Insbesondere bei um 45° geneigten Versteifungselementen kann die Torsionssteifigkeit gegenüber einer herkömmlichen Kolbenstange bei gleichem Gewicht, Biegesteifigkeit und Werkzeugaufwand um einen Faktor 5 erhöht werden. Ein weiterer Vorteil einer solchen 45°-Verstrebung besteht darin, dass die Abstände der Auflage-Rippen am Kolbenstopfende reduziert werden können. Auch kann, da es sich hier um eine einseitige Einspannung handelt, die Materialstärke der Wand in Richtung des zweiten Kolbenstangenendes mit der Druckplatte reduziert werden. Besonders vorteilhaft ist es, wenn die Kolbenstange einen kreuzförmigen Querschnitt umfasst. Besonders hohe Torsionssteifigkeit wird erzielt, wenn die Kolbenstange zusätzlich zu den Versteifungselementen, insbesondere Versteifungsrippen, wenigstens einen rohrartigen Abschnitt umfasst.

Dieser rohrartige Abschnitt kann nicht zusätzlich zu den Versteifungsrippen vorgesehen sein, sondern auch alleine, um die Mittel zur Erhöhung der Biege- und Torsionssteifigkeit bereitzustellen.

Der rohrartige Abschnitt kann als einzelner Abschnitt sich über die ganze Kolbenstange oder einen wesentlichen Teil der Kolbenstange, beispielsweise 30 bis 90 % der Kolbenstange, erstrecken. Alternativ ist es möglich, dass die Kolbenstange nicht nur einen einzigen rohrartigen Abschnitt, sondern mehrere rohrartige Abschnitte oder einen oder mehrere Teilabschnitte eines rohrartigen Abschnittes umfasst. Die Teilabschnitte sind beispielsweise 180°-Abschnitte eines rohrartigen Abschnittes, also Halbrohre. Auch 90°-Abschnitte, also Viertelrohre sind möglich. Der rohrartige Abschnitt weist in einer ersten Ausgestaltung einen Rohrdurchmesser auf, wobei der Rohrdurchmesser beispielsweise ungefähr dem Außendurchmesser der Kolbenstange entsprechen kann oder aber 10 bis 90 % des Außendurchmessers der Kolbenstange beträgt. Zusätzlich zur Versteifung durch die rohrartigen Abschnitte ist es aber auch möglich, eine Versteifung vorzusehen, dadurch, dass sowohl außerhalb und/oder innerhalb des wenigstens einen rohrartigen Abschnitts zusätzliche Versteifungselemente angeordnet sind.

Um ein Verkanten beim Einsetzen der Kolbenstange in den Spritzenkörper zu vermeiden und so ein eventuell nicht erwünschtes Austreten von Medikament aus einem gefüllten Spritzenkörper oder einer Karpule, kann vorgesehen sein, im Bereich des ersten Kolbenstangenendes mit dem Kolbenstopfen Auflageelemente anzuordnen. Betätigt wird der Kolben mit Kolbenstange durch eine am zweiten Kolbenstangenende angeordnete Druckplatte.

Die Torsionssteifigkeit kann man bei der erfindungsgemäßen Kolbenstange durch unterschiedliche Maßnahmen erhöhen. Erfindungsgemäß wird die Torsionssteifigkeit erhöht, wenn man eine bzw. mehrere Querschnittsflächen entfernt zur Torsionsachse bereitstellt. Die Torsionssteifigkeit wird weiter erhöht, wenn Querschnittsfläche möglichst entfernt und in großer Menge zur Torsionsachse zur Verfügung gestellt wird. Eine weitere Erhöhung ist möglich, wenn Querschnittsfläche kontinuierlich über die senkrechte Länge der Torsionsachse verteilt wird. Nähern die kontinuierlich verlaufende Querschnittsflächen sich der Form mehrerer gegenläufig verlaufener Spiralen an, so wird die Torsionssteifigkeit noch weiter erhöht.

Verwendung findet die erfindungsgemäße Kolbenstange in einem Spritzenkörper mit einem Spritzenkonus oder in einer Karpule, wobei im Spritzenkörper oder in der Karpule eine erfindungsgemäße Kolbenstange geführt wird.

Die Erfindung soll nachfolgend anhand der Figuren näher beschrieben werden.

Es zeigen:
- Fig. 1a-1e: eine erste Ausführungsform einer Kolbenstange mit einem rohrförmigen Abschnitt und Versteifungselementen;
- Fig. 2a-2b: einen Vergleich einer erfindungsgemäßen Ausführungsform gemäß Figur 1a-1e mit einer konventionellen Ausführungsform (Fig. 2b);
- Fig. 3a-3e: eine Ausgestaltung einer Stange mit sich über 90 % der Kolbenstange erstreckendem rohrförmigen Element;
- Fig. 4a-4b: eine Kolbenstange gemäß Fig. 3a-3e im Vergleich zu herkömmlicher Kolbenstange gemäß dem Stand der Technik; (Fig.4b);
- Fig. 5a-5e: eine Kolbenstange mit abschnittsweisen rohrförmigen Elementen entlang der Kolbenstange sowie zusätzlichen Versteifungselementen;
- Fig. 6a-6e: eine Kolbenstange mit abschnittsweise rohrförmigen Elementen sowie eine zweite Art von Versteifungselementen in Form von Wabenkörpern.
- Fig. 7a-7b: einen Vergleich einer Kolbenstange gemäß Fig. 5a-5b mit einer Kolbenstange gemäß dem Stand der Technik; (Fig.7b);
- Fig. 8a-8b: einen Vergleich einer Kolbenstange gemäß Fig. 6a-6b mit einer Kolbenstange gemäß dem Stand der Technik; (Fig.8b)
- Fig. 9a-9e: eine Ausführungsform einer Kolbenstange, umfassend nur abschnittsweise schräg gegenüber der Torsionsachse angeordnete Versteifungselemente;
- Fig. 10a-10b: einen Vergleich einer Kolbenstange gemäß Fig. 9a-9e mit einer Kolbenstange gemäß dem Stand der Technik. (Fig.9b)

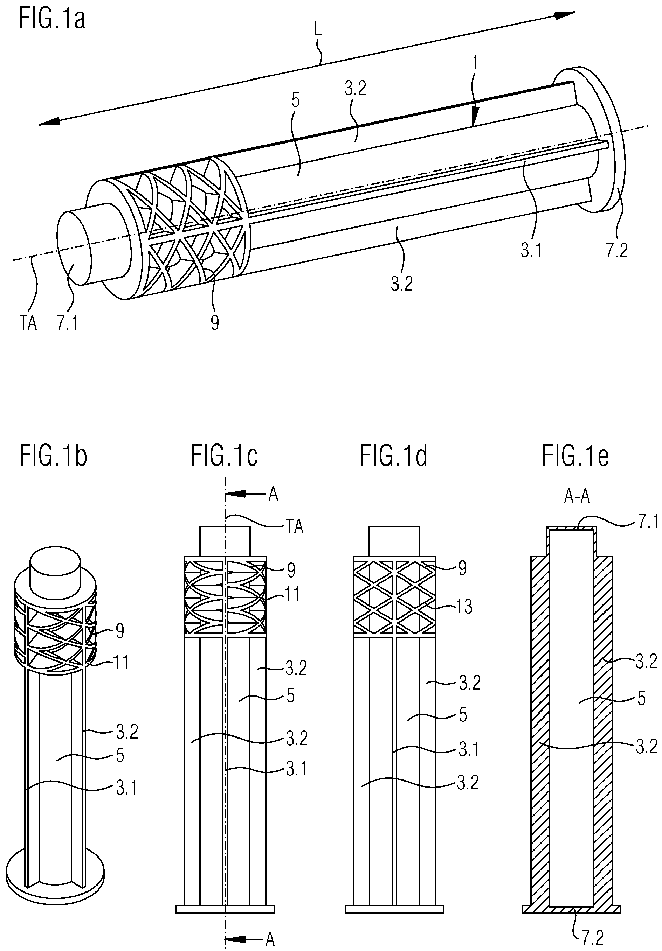

In den Figuren 1a-1e ist eine erste Ausgestaltung einer erfindungsgemäßen Kolbenstange 1 gezeigt, wobei die Kolbenstange im Querschnitt wie im Stand der Technik zwei gekreuzte Rippen 3.1, 3.2 umfasst. Zusätzlich weist die Kolbenstange gemäß dem Ausführungsbeispiel 1a-1e einen rohrförmigen Abschnitt 5 auf. Der rohrförmige Abschnitt erstreckt sich in dem Ausführungsbeispiel gemäß Figuren 1a-1e über praktisch 100% der Länge L der Kolbenstange vom ersten Kolbenstangenende 7.1 zum zweiten Kolbenstangenende 7.2.

Durch den rohrförmigen Abschnitt 5 wird eine Querschnittsfläche entfernt von der Torsionsachse TA zur Verfügung gestellt und so die Torsions- und Biegesteifigkeit der Kolbenstange erhöht. Zusätzlich zu dem rohrförmigen Element 5 sind an der Außenseite des rohrförmigen Elements 5 bei der Ausführungsform gemäß Fig. 1a-1b Versteifungselemente 9 im Bereich des ersten Kolbenstangenendes 7.1 vorgesehen, die zum einen dazu dienen, weitere Querschnittsflächen außerhalb der Torsionsachse zur Verfügung zu stellen sowie im Bereich des ersten Kolbenstangenendes 7.1, das zum Kolbenstopfen korrespondiert, Auflagerippen, die dafür sorgen, dass die Spritze sich nach Einführen in einen Spritzenkörper nicht verkanten kann. Die Figuren 1b und 1c zeigen einen Spritzenkörpers gemäß Fig. 1a, im Detail und unterschiedlichen Ansichten, wobei gleiche Bauteile mit denselben Bezugsziffern belegt sind. Bei einem Spritzgusswerkzeug entspricht die Entformungsrichtung der Prägerichtung. Die Prägerichtung zeigt von der Werkzeugkernseite zur Werkzeugdüsenseite.

Fig. 1d zeigt die gekreuzte Anordnung der Verstärkungselemente 9. Fig. 1e ist ein Schnitt durch die Ausgestaltung gemäß Fig. 1c entlang der Linie A-A und zeigt deutlich den rohrförmigen Abschnitt 5 im Querschnitt. Gut zu erkennen ist das erste Kolbenstangenende 7.1 gleichbedeutend mit dem Stopfen und das zweite Kolbenstangenende 7.2, das gleichzusetzen ist mit einer Druckplatte, die dazu dient, die Kolbenstange in axialer Richtung zu verschieben Des Weiteren aus Figur 1e zu entnehmen sind die Rippen 3.2 und die Tatsache, dass es sich bei dem rohrförmigen Abschnitt um einen Hohlkörper handelt. Ein derartiger komplett geschlossener Hohlkörper kann nur mit speziellen Verfahren hergestellt werden, beispielsweise einem Gasinnendruck-Verfahren. Alternativ kann man einen Hohlkörper auch mit Hilfe eines Spritzguss-Werkzeuges mit langem Kern erhalten, den man von der Kolbenstopfseite in die Kolbenstange einführt. Das sich dann ergebende Rohr ist dann nur einseitig geschlossen.

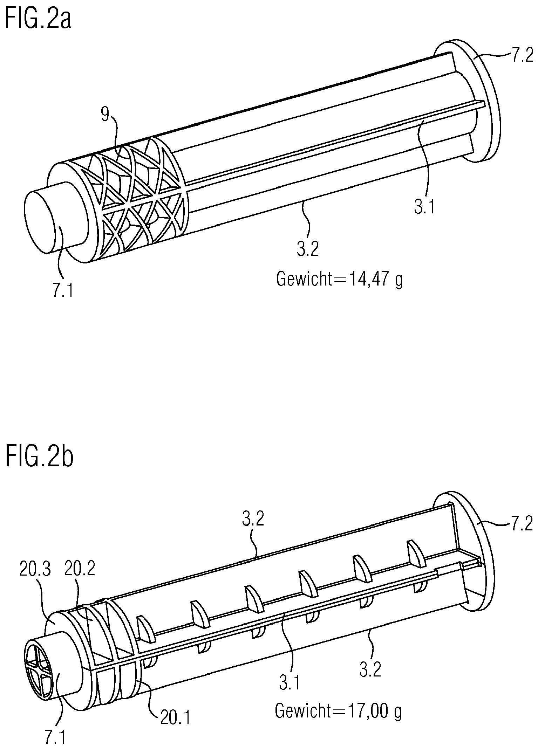

Die Figuren 2a und 2b zeigen eine Gegenüberstellung der erfindungsgemäßen Kolbenstange gemäß Fig. 1a, verglichen mit einer Kolbenstange gemäß dem Stand der Technik. Die Kolbenstange gemäß Fig. 2a entspricht der Kolbenstange gemäß Fig. 1a. Gleiche Bauteile wie in Fig. 1a sind mit denselben Bezugsziffern belegt. Fig. 2b zeigt eine Kolbenstange gemäß dem Stand der Technik mit gekreuzten Rippen 3.1, 3.2. Das erste Kolbenstangenende mit dem Kolbenstopfen ist mit 7.1 bezeichnet, das zweite Kolbenstangenende mit der Druckplatte mit 7.2.

Um ein Verkanten der Kolbenstange wie im Stand der Technik zu vermeiden, sind, wie beispielsweise in der US 7,824,380 B2 gezeigt, im Bereich des ersten Kolbenstangenendes beim Stand der Technik gemäß Fig. 2b Scheiben 20.1, 20.2, 20.3 vorgesehen.

Das Gewicht der Kolbenstange gemäß dem Stand der Technik beträgt 17 g, das Gewicht der Kolbenstange gemäß Fig. 2a und 1a 14,5 g. Die erfindungsgemäße Kolbenstange gemäß Fig. 2a und Fig. 1a weist eine um einen Faktor 11 kleinere Torsionsspannung als die Ausgestaltung gemäß dem Stand der Technik (Fig. 2b) auf sowie eine um einen Faktor 16 kleinere Torsionsverschiebung gegenüber der Ausgestaltung gemäß dem Stand der Technik (Fig. 2b).

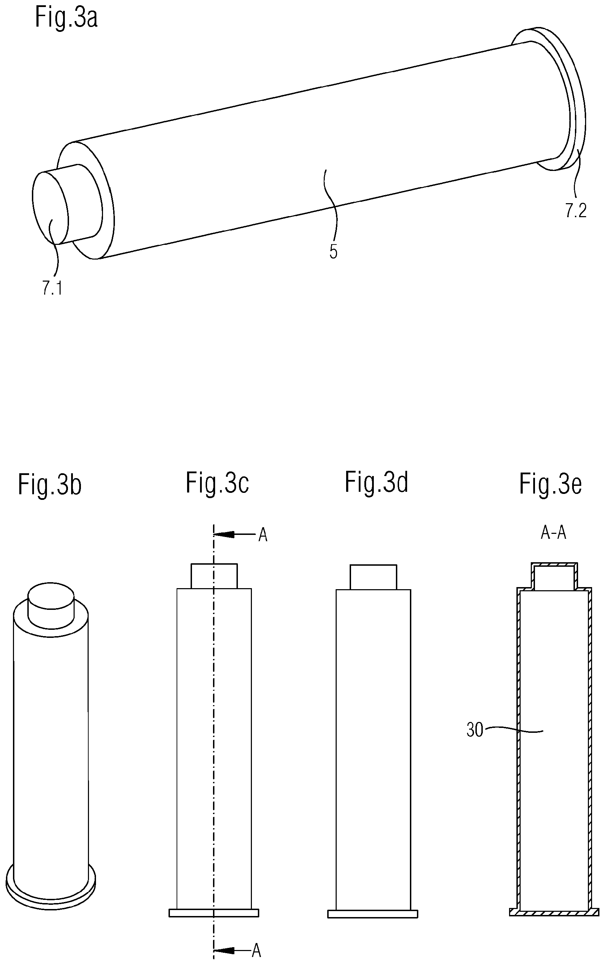

In den Figuren 3a-3e ist eine alternative Ausgestaltung einer Kolbenstange mit rohrförmigem Körper dargestellt. Der rohrförmige Körper 5 weist einen Durchmesser auf, der im Wesentlichen dem Durchmesser der Kolbenstange entspricht. Das erste Kolbenstangenende, umfassend den Kolbenstopfen, ist mit 7.1, das zweite Kolbenstangenende mit 7.2 bezeichnet.

Bei der Ausgestaltung gemäß Fig. 3a sind keine zusätzlichen Verstärkungselemente wie bei der Ausführungsform in den Figuren 1a-1e vorgesehen. Die Verkippung beim Einführen des Spritzenkolbens in die Spritze wird dadurch vermieden, dass der rohrförmige Körper im Wesentlichen denselben Außendurchmesser aufweist wie der Spritzenkörper selbst. Die Figuren 3b bis 3e sind alternative Ansichten inklusive Schnittansicht A-A gemäß Fig. 3e. Gleiche Bauteile sind mit denselben Bezugsziffern wie in Fig. 3a belegt. Wie aus Fig. 3e deutlich zu erkennen ist der rohrförmige Körper innen hohl, d.h. er weist einen Hohlraum 30 auf. Die Ausgestaltung gemäß Fig. 3a-3e zeigt eine besonders bevorzugte Ausgestaltung einer Kolbenstange. Nachteilig an der Ausgestaltung gemäß Fig. 3a-3e ist lediglich die aufwändige Herstellung, insbesondere der hohe Herstellaufwand für den Hohlkörper. Ein mögliches Herstellverfahren ist der 3D-Druck. Die Ausgestaltung der Kolbenstange gemäß Fig. 1a-e und Fig. 2a ist einfacher herstellbar als ein kompletter Hohlkörper, da die in der Ausführungsform gemäß Fig. 1a-e vorgesehenen Verrippungen einen geringeren Werkzeugaufwand erzeugen.

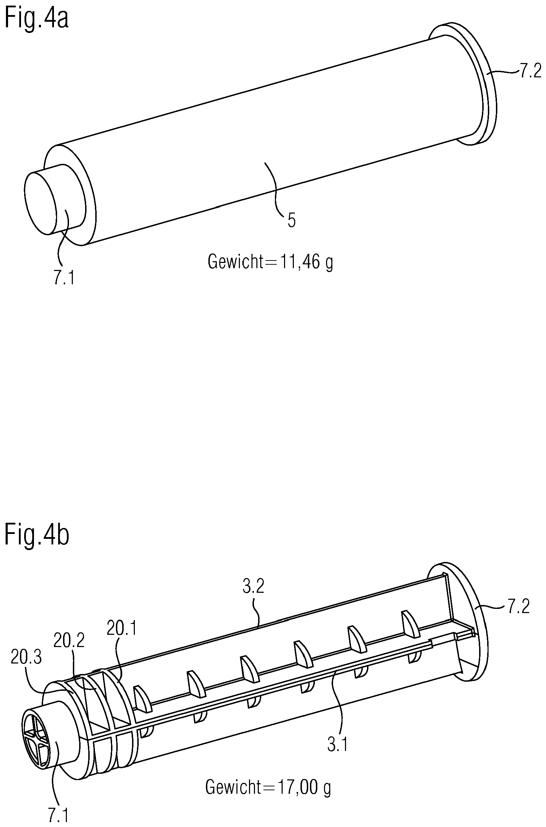

In den Figuren 4a und 4b ist nochmals die erfindungsgemäße Kolbenstange gemäß Fig. 3a im Vergleich zu einer Kolbenstange gemäß dem Stand der Technik (Figur 4b) gezeigt. Die Kolbenstange gemäß dem Stand der Technik (Figur 4 b) entspricht derjenigen in Fig. 2b. Insoweit wurden die Bezugsziffern aus Fig. 2b in Fig. 4b übernommen. Das Gewicht der Kolbenstange gemäß Fig. 4b beträgt 17 g, das Gewicht der erfindungsgemäßen Kolbenstange gemäß Fig. 4a aufgrund der Tatsache, dass der rohrförmige Körper als Hohlkörper ausgebildet ist, nur 11,46 g. Gegenüber dem herkömmlichen Bauteil gemäß Fig. 4b weist die erfindungsgemäße Kolbenstange gemäß Fig. 4a eine um einen Faktor 11 größere Torsionsspannung als das Bauteil gemäß dem Stand der Technik auf sowie eine um einen Faktor 55 größere Torsionsverschiebung

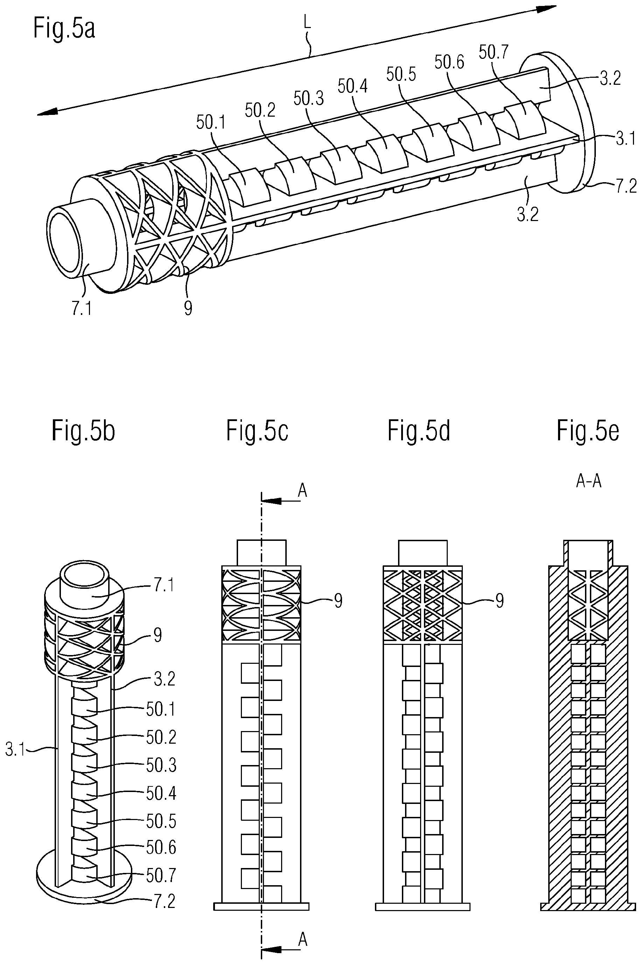

In den Fig. 5a-5e ist eine alternative Ausgestaltung einer erfindungsgemäßen Kolbenstange gezeigt. Die Kolbenstange umfasst entlang ihrer Länge L nicht einen einzigen rohrförmigen Abschnitt, sondern eine Vielzahl von rohrförmigen Abschnitten 50.1, 50.2, 50.3, 50.4, 50.5, 50.6, 50.7., die vorliegenden Teilabschnitte d.h. 90°-Abschnitte eines rohrartigen Abschnittes. Die rohrförmigen 90°-Abschnitte sind in der dargestellten Ausführungsform somit Viertelrohre. Möglich wären auch Halbrohre, d. h. 180°-Abschnitte, allerdings sind 90°-Abschnitte effektiver. Dies ist darauf zurückzuführen, dass eine Idealstruktur eines auf Torsionslast ausgelegten Rohres mit offenem Rohrmantel mehrere gegenläufige Spiralen umfasst. Eine Kolbenstange mit 90°-Abschnitten hat aufgrund dieser Erkenntnis einen idealeren, gleichmäßigen Kraftfluss und höhere Steifheit als 180°-Abschnitte. Bei der Ausgestaltung gemäß Fig. 5a-5e gelingt es bei einer Kolbenstange, die in einem Standard-Spritzguss-Verfahren hergestellt wird, die Torsionssteifigkeit zu erhöhen. Wie schon bei der Ausgestaltung gemäß Fig. 1a sind zusätzliche Versteifungselemente 9 im Bereich des ersten Kolbenstangenendes 7.1 vorgesehen. Diese sind wie in Fig. 1a bis 1e kreuzförmig ausgebildet. Das zweite Kolbenstangenende 7.2 ist wiederum als Druckplatte ausgeführt. Weiter kann man am Kolbenstangenende 7.1 eine stirnseitig Öffnung erkennen sowie einen Hohlraum unter den dortigen Versteifungsrippen. Fig. 5a bis 5e zeigen in einer ersten Ausgestaltung eine Kolbenstange mit abschnittsweisen rohrförmigen Elementen 50.1, 50.2, 50.3, 50.4, 50.5, 50.6 sowie 50.7. als Viertelrohren. Die zusätzlichen Verstärkungselemente sind mit 9 gekennzeichnet und im Bereich des ersten Kolbenstangenendes angeordnet. Fig. 5d ist ein Schnitt gemäß Schnittlinie A-A in Fig. 5c. Deutlich zu erkennen sind die einzelnen rohrförmigen Teilabschnitte 50.1, 50.2, 50.3, 50.4, 50.5 und 50.6 sowie die gekreuzten Versteifungselemente.

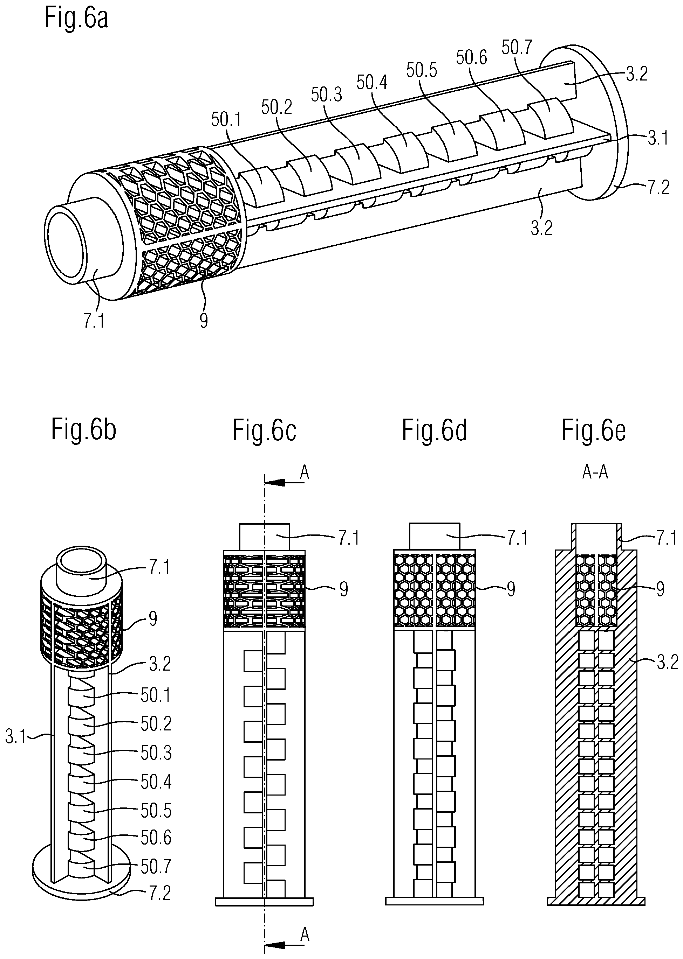

In Fig. 6a bis 6e ist eine alternative Ausgestaltung zu Fig. 5a bis 5e gezeigt, ebenfalls mit rohrförmigen Teilabschnitten, wobei es sich bei den Teilabschnitten wieder um 90°-Abschnitte, d.h. Viertelrohre handelt. Im Gegensatz zu den Fig. 5a bis 5e sind aber die zusätzlichen Verstärkungselemente anders ausgestaltet. So weisen die zusätzlichen Verstärkungselemente 9 bei der Ausführungsform gemäß den Fig. 6a bis 6e eine wabenförmige Form auf, die rohrförmigen Teilabschnitte 50.1, 50.2, 50.3, 50.4 50.5, 50.6, 50.7 sind, wie in Fig. 6a, versetzt zueinander angeordnet. Prinzipiell stellen die Rohrabschnitte selbst auch Verstärkungselemente dar. Die als Waben ausgebildeten zusätzlichen Verstärkungselemente haben den Vorteil, dass der mögliche Verkippwinkel der Kolbenstange in dem Spritzenkörper reduziert wird, da die Spritzenkörperkante beim Verkippen schneller auf eine Verrippung trifft, da die Bereiche in denen keine Verrippung vorliegt viel kleiner werden. Dieser positive Effekt liegt ebenso bei einer schrägen (z.B.45°) Verrippung vor. Eine gleichwertige Reduktion des Verkippwinkels kann mit einfachen Tellern nicht erreicht werden.

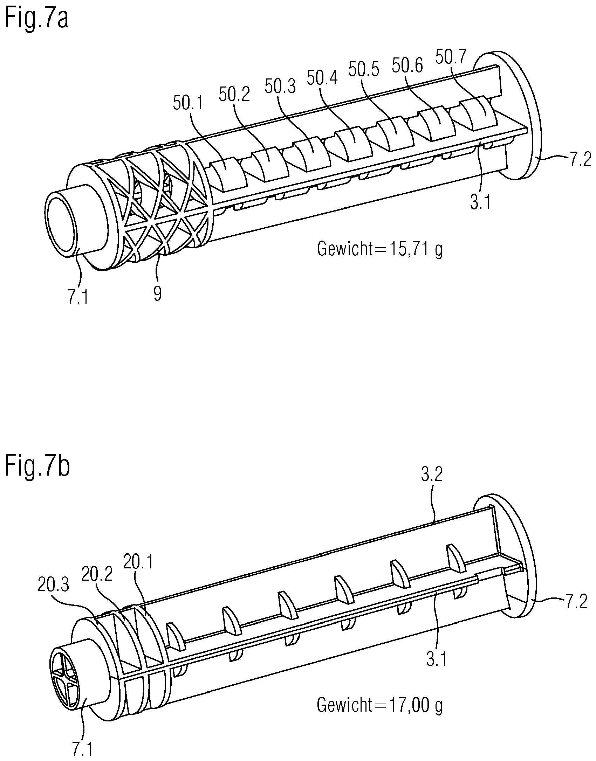

Die Figuren 7a und 7b zeigen in einer Gegenüberstellung eine erfindungsgemäße Kolbenstange mit rohrförmigen Abschnitten gemäß Fig. 5a im Vergleich zu einer herkömmlichen Kolbenstange gemäß Fig. 7b. Das Gewicht der erfindungsgemäßen Kolbenstange beträgt 15,71 g, das Gewicht der herkömmlichen Kolbenstange 17 g. Die Torsionsspannung der erfindungsgemäßen Kolbenstange ist um einen Faktor 3 kleiner als derjenige der Referenzkolbenstange gemäß Fig. 7b. Die Torsionsverschiebung ist um einen Faktor 7 geringer.

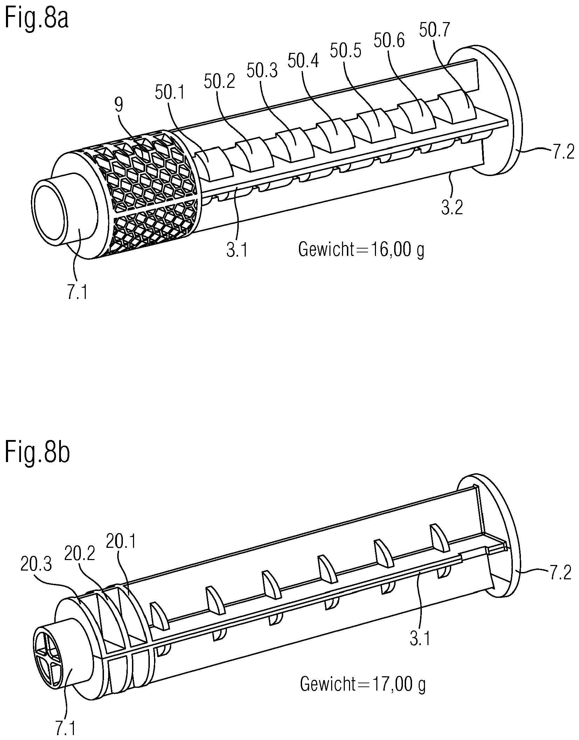

In den Figuren 8a und 8b ist eine erfindungsgemäße Kolbenstange gemäß Figur 6a einer herkömmlichen Kolbenstange gemäß Fig. 8b gegenübergestellt. Das Gewicht der erfindungsgemäßen Kolbenstange beträgt 16 g, das der herkömmlichen Kolbenstange 17 g. Die Torsionsverschiebung der erfindungsgemäßen Kolbenstange ist um einen Faktor 4 kleiner als diejenige der Kolbenstange gemäß dem Stand der Technik.

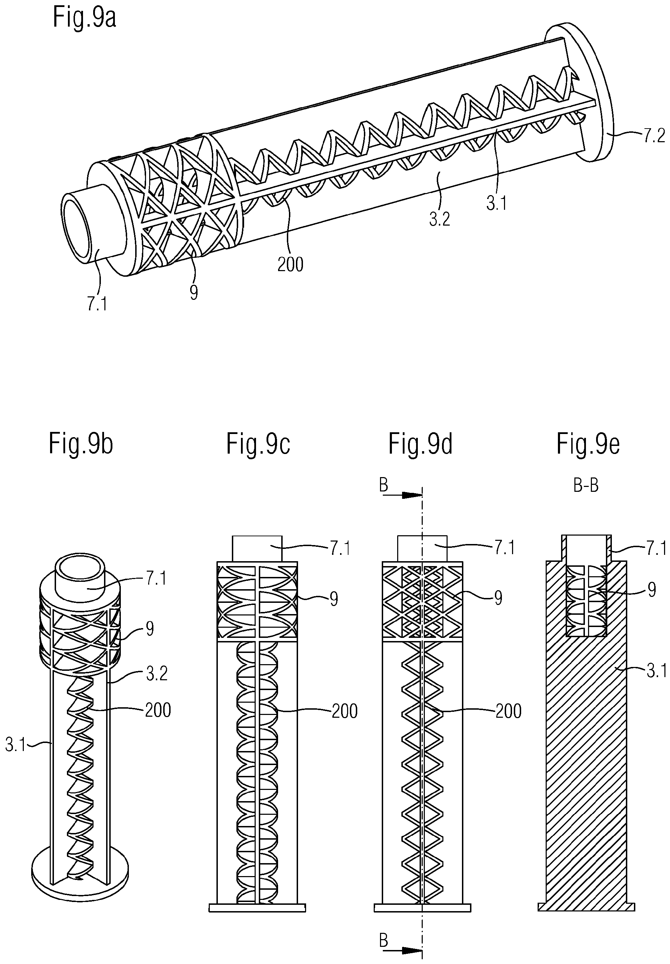

In Fig. 9a ist eine gänzlich andere Ausgestaltung einer erfindungsgemäßen Kolbenstange gezeigt. Die Ausführungsform gemäß Fig. 9a umfasst keine rohrförmigen Abschnitte, sondern anstelle des oder der rohrförmigen Abschnitte Verstrebungen 200, die bevorzugt kreuzförmig wie die Versteifungselemente im vorderen Abschnitt der Kolbenstange ausgeführt sind. Die Versteifungselemente im vorderen Kolbenstangenabschnitt sind wie in Fig. 1a schräg zur Torsionsachse ausgeführt und dienen dazu, ein Verkanten der Spritze zu verhindern. Das vordere Kolbenstangenende 7.1 stellt einen Abschnitt dar, der ein Gewinde aufweist. Das Gewinde wiederum nimmt einen Kolbenstopfen auf. Die Verbindung Kolbenstopfen und Kolbenstange kann auch über einen Hinterschnitt hergestellt werden. Das hintere Kolbenstangenende 7.2 ist als Druckplatte ausgebildet. Die Verstrebungen 200 anstelle des rohrförmigen Abschnittes stellen bei der Ausgestaltung gemäß Figur 9a die Querschnittsfläche außerhalb der Torsionsachse zur Erhöhung der Torsions- bzw. Biegesteifigkeit zur Verfügung im hinteren Abschnitt der Kolbenstange zum hinteren Kolbenstangenende 7.2 zur Verfügung.

In den Figuren 9b bis 9e sind Detailansichten der Kolbenstange gemäß Fig. 9a gezeigt. Gleiche Bauteile wie in Fig. 9a sind mit denselben Bezugsziffern belegt. Fig. 9e zeigt einen Schnitt entlang der Linie B-B aus Fig. 9d. In Fig. 9d ist deutlich die Anordnung der Versteifungsrippen 200 zur Erhöhung der Torsions- und Biegesteifigkeit der Kolbenstange gezeigt.

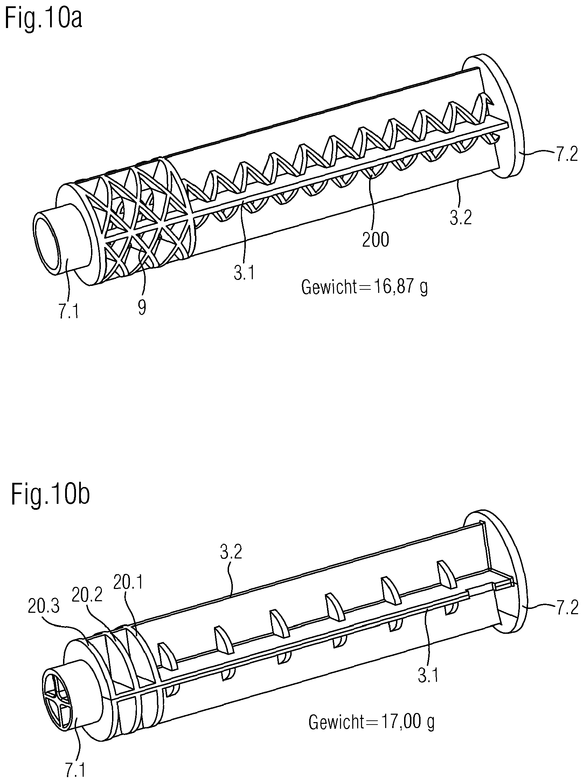

Fig. 10a und 10b zeigen wiederum eine Gegenüberstellung der erfindungsgemäßen Kolbenstange gemäß Fig. 9a mit einer Kolbenstange gemäß dem Stand der Technik in Fig. 10b. Die Kolbenstange gemäß dem Stand der Technik hat ein Gewicht von 17 g, die erfindungsgemäße Kolbenstange gemäß Fig. 10a von 16,87 g. Die Torsionsspannung der erfindungsgemäßen Kolbenstange ist um einen Faktor 2 kleiner als diejenige des Standes der Technik, die Torsionsverschiebung um einen Faktor 4.

Um das Gewicht der Kolbenstange gering zu halten besteht die Kolbenstange bevorzugt aus einem Kunststoffmaterial. Die aus Kunststoff bestehende Kolbenstange kann sowohl in einer Kunststoffspritze als auch einer Glasspritze verwandt werden.

Besonders bevorzugte Kunststoffmaterialien sind thermoplastische Kunststoffe. Ein möglicher thermoplastischer Kunststoff kann Polyethylen (PE) oder Polyoxymethylen (POM) oder Polystyrol (PS) sein. Ein anderes Polymer kann Polypropylen (PP) sein. Harte Polymere, wie Cycloolefincopolymer (COC) oder Cycloolefinpolymere (COP) finden insbesondere als Materialien für den Spritzenkörper Verwendung. Um den Spritzenkörper zur Lagerung pharmazeutischer Produkten einsetzen zu können, sind die Materialien des Spritzenkörpers bei Temperaturen >100°C, insbesondere 121°C, insbesondere maximal 180°C steriliserbar. Während Cycloolefinpolymere (COP) und Cycloolefincopolymere (COC) harte Materialien sind in denen pharmazeutische Medien über mehrere Monate gelagert werden können, ist Polypropylen oder Polyethylen ein weicheres Material, das Spannungen aufnehmen kann, aber aufgrund seines Diffusionsverhaltens eine Lagerung von pharmazeutischen Produkten über einen längeren Zeitraum, wenn es als Material für den Spritzenkörper verwandt wird, nicht zulässt.

Wird die Kolbenstange aus einem thermoplastischen Material als Hohlkörper ausgebildet, so kann die mit Hilfe eines Spritzgieß-Verfahrens hergestellte Kolbenstange mit Hilfe eines Spritzguss-Werkzeuges mit einem langen Kern erhalten werden, den man beispielsweise von der Kolbenstopfenseite in die Kolbenstange einführt. Eine sich dann ergebende Kolbenstange ist nur einseitig geschlossen. Alternativ könnte auch mit Hilfe eines Gasinnendruck-Verfahrens als speziellem Spritzgussverfahren aus einem Kunststoffmaterial ein geschlossener Hohlkörper erhalten werden.

Durch die Verwendung eines Kunststoffmaterials anstelle eines Glasmaterials wie in der US 2,392,104 B offenbart können erhebliche Gewichtsreduktionen erreicht werden, insbesondere wenn die Kolbenstange als Kunststoffteil ausgeführt ist.

Mit der Erfindung werden erstmals Ausgestaltungen von Kolbenstangen angegeben, die eine erhöhte Torsions- und Biegesteifigkeit gegenüber herkömmlichen Kolbenstangen wie im Stand der Technik, beispielsweise in der US 7,824,380, aufweisen. Des Weiteren wird einem Verkippen der Kolbenstange im Spritzenkörper entgegen gewirkt.

## Patentansprüche

1. Kolbenstange, bevorzugt hohle Kolbenstange für medizinische Packmittel, insbesondere Spritzenkörper, Karpulen wobei, die Kolbenstange ein erstes und ein zweites Kolbenstangenende (7.1, 7.2) umfasst,
**dadurch gekennzeichnet, dass**
die Kolbenstange (1) eine Torsionsachse (TA) und Querschnittsflächen umfasst sowie Mittel zur Erhöhung der Biege- und Torsionssteifigkeit
**dadurch gekennzeichnet, dass**
die Mittel zur Erhöhung der Biege- und Torsionssteifigkeit, Bestandteile der Kolbenstange, die wenigstens eine Querschnittsfläche entfernt zur Torsionsachse zur Verfügung stellen, sind.

2. Kolbenstange nach Anspruch 1,
**dadurch gekennzeichnet, dass**
die Kolbenstange mit Hilfe eines Spritzgussverfahrens erhalten wird.

3. Kolbenstange nach Anspruch 1 oder 2,
**dadurch gekennzeichnet, dass**
die Kolbenstange aus einem Kunststoffmaterial, insbesondere einem Thermoplast, bevorzugt einem der der folgenden Materialien besteht:
- Polypropylen (PP)
- Polystyrol (PS)
- Polyethylen (PE)
- Polyoxymethylen (POM)

4. Kolbenstange nach einem der Ansprüche 1 bis 3,
**dadurch gekennzeichnet, dass**
die Kolbenstange (1) wenigstens abschnittsweise schräg gegenüber der Torsionsachse angeordnete Versteifungselemente (200), insbesondere Versteifungsrippen als Mittel zur Erhöhung der Biege- und Torsionssteifigkeit, umfasst.

5. Kolbenstange nach einem der Ansprüche 1 bis 4,
**dadurch gekennzeichnet, dass**
die Versteifungselemente (200), insbesondere Versteifungsrippen, gegenüber der Torsionsachse um einen Winkel α, der bevorzugt größer als 10°, insbesondere größer als 20°, bevorzugt größer als 30°, insbesondere 45°, geneigt ist.

6. Kolbenstange nach einem der Ansprüche 1 bis 5,
**dadurch gekennzeichnet, dass**
die Kolbenstange (1) einen kreuzförmigen Querschnitt (3.1, 3.2) aufweist.

7. Kolbenstange nach einem der Ansprüche 1 bis 3,
**dadurch gekennzeichnet, dass**
die Kolbenstange als Mittel zur Erhöhung der Biege-und Torsionssteifigkeit wenigstens einen rohrartigen Abschnitt (5) oder Teilabschnitt, insbesondere einen hohlen rohrartigen Abschnitt oder Teilabschnitt umfasst.

8. Kolbenstange nach Anspruch 7,
**dadurch gekennzeichnet, dass**
die Kolbenstange des Weiteren wenigstens abschnittsweise, bevorzugt schräg gegenüber der Torsionsachse angeordnete Versteifungselemente, insbesondere Versteifungsrippen umfasst.

9. Kolbenstange nach Anspruch 7 oder 8,
**dadurch gekennzeichnet, dass**
die Kolbenstange mehrere rohrartige Abschnitte (50.1, 50.2, 50.3, 50.4, 50.5, 50.6, 50.7) oder Teilabschnitte umfasst.

10. Kolbenstange nach einem der Ansprüche 7 bis 9,
**dadurch gekennzeichnet, dass**
der wenigstens eine rohrartige Abschnitt oder Teilabschnitt einen Rohrdurchmesser aufweist, wobei der Rohrdurchmesser ungefähr mit dem Außendurchmesser der Kolbenstange übereinstimmt oder
der wenigstens eine rohrartige Abschnitt einen Rohrdurchmesser aufweist, wobei der Rohrdurchmesser 10 bis 90 % des Außendurchmessers der Kolbensstange beträgt.

11. Kolbenstange nach einem der Ansprüche 7 bis 10,
**dadurch gekennzeichnet, dass**
außerhalb und/oder innerhalb des wenigstens einen rohrartigen Abschnittes zusätzliche Verstärckungselemente (9) angeordnet sind, wobeidie zusätzlichen Verstärkungselemente (9) bevorzugt kreuzförmig und/oder wabenförmig ausgebildet sind.

12. Kolbenstange nach einem der Ansprüche 1 bis 11,
**dadurch gekennzeichnet, dass**
die im Bereich des ersten Kolbenstangenendes (7.1) mit einem Kolbenstopfen versehenen Auflageelemente angeordnet sind.

13. Kolbenstange nach einem der Ansprüche 1 bis 12,
**dadurch gekennzeichnet, dass**
das zweite Kolbenstangenende (7.2) eine Druckplatte umfasst.

14. Spritzenkörper mit einem Spritzenkonus,
**dadurch gekennzeichnet, dass**
der Spritzenkörper eine im Spritzenkörper geführte Kolbenstange gemäß einem der Ansprüche 1 bis 13 umfasst.

15. Karpule, **dadurch gekennzeichnet, dass** die Karpule eine in der Karpule geführte Kolbenstange gemäß einem der Ansprüche 1 bis 13 umfasst.
